# EUROPEAN PATENT SPECIFICATION

(11) **EP 1 367 988 B1**
(45) Date of publication and mention of the grant of the patent: **28.02.2007**
(21) Application number: 01992561.9
(22) Date of filing: 24.10.2001
(51) Int. Cl.: A61K 8/97, A61Q 19/08

(54) **SKIN TREATMENT BASED ON THE USE OF CHROMOLAENA ODORATA**
METHOD ZUR BEHANDLUNG DER HAUT BASIEREND AUF DER VERWENDUNGVON CHROMOLAENA ODORATA
PROCEDE DE TRAITEMENT DE LA PEAU BASES SUR L'UTILISATION DE LA CHROMOLAENA ODORATA

(30) Priority: 02.11.2000 GB 0026828
(43) Date of publication of application: 10.12.2003
(60) Divisional application: 06001811.6
(73) Proprietor: UNILEVER PLC, London EC4P 4BQ (GB); UNILEVER N.V., 3013 AL Rotterdam (NL)
(72) Inventor: DONOVAN, Robert Mark,, 95160 Montmorency (FR)
(74) Representative: Acham, Nicholas Clive
(86) International application number: PCT/EP2001/012387
(87) International publication number: WO 2002/036091

(56) References cited:
- WO-A-01/08653
- PHAN T T ET AL: "ENHANCED PROLIFERATION OF FIBROBLASTS AND ENDOTHELIAL CELLS TREATEDWITH AN EXTRACT OF THE LEAVES OF CHROMOLAENA ODORATA (EUPOLIN), ANDHERBAL REMEDY FOR TREATING WOUNDS" PLASTIC AND RECONSTRUCTIVE SURGERY, WILLIAMS AND WILKINS CO., BALTIMORE, MD, US, vol. 101, no. 3, 1998, pages 756-765, XP001057042 ISSN: 0032-1052 cited in the application
- PHAN T. T. ET AL: "AN AQUEOUS EXTRACT OF THE LEAVES OF CHROMOLAENA ODORATA (FORMELY EUPATORIUM ODORATUM) (EUPOLIN) INHIBITS HYDRATED COLLAGEN LATTICE CONTRACTION BY NORMAL HUMAN DERMAL FIBROPLASTS" THE JOURNAL OF ALTERNATIVE AND COMPLIMENTARY MEDICINE, vol. 2, no. 3, 1996, pages 335-343, XP001057476 cited in the application
- PHAN T-T ET AL: "PHENOLIC COMPOUNDS OF CHROMOLAENA ODORATA PROTECT CULTURED CKIN CELLS FRM OXIDATIVE DAMAGE: IMPLICATION FOR CUTANEOUS WOUND HEALING" BIOLOGICAL & PHARMACEUTICAL BULLETIN (OF JAPAN), PHARMACEUTICAL SOCIETY OF JAPAN, JP, vol. 24, no. 12, December 2001 (2001-12), pages 1373-1379, XP001061664 ISSN: 0918-6158

## Description

This invention relates to a cosmetic method of improving the condition and appearance of skin, and to the use of a particular plant extract in the preparation of topical compositions for improving the condition and appearance of skin. It relates to the use of extracts of Chromolaena Odorata (also known for example as Siam Weed or Devils Weed) for the treatment of wrinkles and/or photodamaged skin.

Skin is subject to deterioration through dermatological disorders, environmental abuse (wind, air conditioning, central heating) or through the normal ageing process (chronoageing) which may be accelerated by exposure of skin to sun (photoageing). In recent years the demand for cosmetic methods for improving the appearance and condition and, in particular, for reversing, reducing or preventing the visible signs of wrinkled, aged and/or photodamaged skin has grown enormously.

Consumers are increasingly seeking "anti-ageing" cosmetic products that reverse, treat or delay the visible signs of chronoageing and photoageing skin such as wrinkles, lines, sagging, hyperpigmentation and age spots.

Collagen, the predominant matrix skin protein is known to impart tensile strength to skin. It is also known in the art that the levels of collagen in skin are significantly reduced with aged and/or photodamaged skin. Many studies have shown that the levels of collagen type I in skin is decreased with age and/or with increased photodamage, (see for example Lavker, R. J. Inv.Derm., (1979), 73,79-66; Griffiths et al. N. Eng. J. med. (1993) 329, 530-535). The reduction of the levels of collagen in skin is accordingly associated with a decrease in the tensile strength of the skin, causing wrinkles and laxity. It is widely accepted that strengthening of the dermal matrix by boosting the levels of collagen in skin provides anti-ageing and dermal repair benefits. It is also known, for example from WO 99/47110, that decorin can act as a marker of skin repair.

Siam weed extract comprising extract of Chromolaena Odorata is a known skin treatment extract, which has found use in the treatment of soft tissue wounds, bums and skin infections, for example in Vietnam. Typical of prior art demonstrating this is "The Journal of Alternative and Complimentary Medicine", Vol. 2, No. 3, 1996 pp 335-343, Phan et al. This describes the use of the aqueous extract of Siam weed leaves for the above mentioned purposes, and the effect of Siam weed extract (termed as Eupolin by Phan et al.) on hydrated collagen lattice contraction by human dermal fibroblasts. Previously prepared petrolatum based Siam weed ointments have been found to contain flavonoids (salvigenin, sakuranetin, isosakuranetin, kaemferid, betulenol, 2-5-7-3-tetra-O-methylquercatagetin, tamarixetin, chalcone 1 and chalcane 2), essential oils (geyren, bornyl acetate, beta eubeden), saponin triterpenoids, tannins, organic acids, and trace elements. In vitro levels of Siam weed extract in topically used compositions were typically 50-200 µg/ml.

In addition, in "Plastic and Reconstructive Surgery", March 1998, "Enhanced proliferation of Fibroplasts and Endothelial Cells Treated with an Extract of the Leaves of Chromolaena Odorata (Eupolin), a Herbal Remedy for Treating Wounds", Phan et al., there is described the use of Siam weed extract to increase fibroblast and endothelial cell growth, again in the context of burns treatment. In particular, it was found that where skin grafts are used, the use of Siam weed extract speeds up the formation of granulation tissue, as well as increasing the rate of collagen synthesis. In this study Siam weed extract ointment was used at topical concentrations of 10-230 µg/ml.

We have surprisingly found that effective treatment and prevention of normal skin conditions due to chronoageing or photoageing, such as wrinkles, lines, sagging, hyperpigmentation and age spots may be obtained through the topical application of cosmetic compositions containing extracts of Chromolaena Odorata.

Thus there is provided a cosmetic method according to the claims appended herein.

Preferably, topical compositions for use in the method according to the invention comprise extract of Chromolaena Odorata at a level of 0.00005 to 10 % of the topical composition, more preferably levels of 0.0001 to 1.0%, and in certain circumstances most preferably 0.001 to 0.1% by weight of the topical composition.

Conveniently, the topical product may be in the form of an oil-in-water emulsion.

In relation to the repair of photodamaged skin, it is generally accepted that the biochemical and biological processes associated with wound repair and remodelling of the dermis are not the same as those involved in photodamage repair. For example, other materials such as marigold and myrrh extracts, which induce wound repair in skin, are known not to be effective at photodamage repair.

The inventive compositions, methods and uses thus provide anti-ageing benefits which may result in the promotion of smooth and supple skin with improved elasticity and a reduced or delayed appearance of wrinkles and aged skin, with improved skin colour. A general improvement in the appearance, texture and condition, in particular with respect to the radiance, clarity, and general youthful appearance of skin may also be achieved. The invention may have particular utility for providing skin care benefits.

The term "treating" as used herein includes within its scope reducing, delaying and/or preventing the above mentioned normal but cosmetically undesirable skin conditions caused by the normal aging process. The visible signs of ageing such as wrinkles, lines and/or sagging may be delayed or reduced. Generally, the quality of skin may be enhanced and its appearance and texture may be improved by preventing or reducing wrinkling and increasing flexibility, firmness, smoothness, suppleness and elasticity of the skin.

The compositions, methods and uses according to the invention may be useful for treating skin that is already in a wrinkled, aged, and/or photodamaged condition, or for treating youthful skin to prevent or reduce those aforementioned undesirable changes due to the normal ageing/photoageing process.

The composition used according to the invention also comprises a dermatologically/cosmetically acceptable vehicle to act as a dilutant, dispersant or carrier for the actives. The vehicle may comprise materials commonly employed in skin care products such as water, liquid or solid emollients, silicone oils, emulsifiers, solvents, humectants, thickeners, powders, propellants and the like.

The vehicle will typically form from 5% to 99.9%, preferably from 25% to 80% by weight of the composition, and can, in the absence of other cosmetic adjuncts, form the balance of the composition.

Besides the extract of Chromolaena Odorata active, other specific skin-benefits actives such as sunscreens, skin-lightening agents, and skin tanning agents may also be included. The vehicle may also further include adjuncts such as antioxidants, perfumes, opacifiers, preservatives, colourants and buffers.

To prepare the topical composition used in the method of the present invention, the usual manner for preparing skin care products may be employed. The active components are generally incorporated in a dermatologically/cosmetically acceptable carrier in conventional manner. The active components can suitably first be dissolved or dispersed in a portion of the water or another solvent or liquid to be incorporated in the composition. The preferred compositions are oil-in-water or water-in-oil or water-in-oil-in-water emulsions, though under certain circumstances water in oil compositions are preferred.

The composition may be in the form of conventional skin-care products such as a cream, gel or lotion, capsules or the like. The composition can also be in the form of a so-called "wash-off" product e.g. a bath or shower gel, possibly containing a delivery system for the actives to promote adherence to the skin during rinsing. Most preferably the product is a "leave-on" product, that is a product to be applied to the skin without a deliberate rinsing step soon after its application to the skin.

The composition may be packaged in any suitable manner such as in a jar, a bottle, tube, roll-ball, or the like, in the conventional manner. A further envisaged form is as a formulation suitable for delivery as a spray, either from a propellant driven aerosol or from a pump spray.

The composition used according to the invention may also be formulated into a form suitable for oral ingestion such as a capsule, tablet or similar.

The method of the present invention may be carried out one or more times daily to the skin which requires treatment. The improvement in skin appearance will usually become visible after 3 to 6 months or possible sooner, depending on skin condition, the concentration of the active components used in the inventive method, the amount of composition used and the frequency with which it is applied. In general, a small quantity of the composition, for example from 0.1 to 5 ml is applied to the skin from a suitable container or applicator and spread over and/or rubbed into the skin using the hands or fingers or a suitable device. A rinsing step may optionally follow, depending on whether the composition is formulated as a "leave-on" or a "rinse-off" product.

In order that the present invention may be more readily understood, the following examples are provided by way of illustration only,

### EXAMPLES

It is known from our co-pending European application no. 99908956.8 that topical retinoic acid treatments can be used to cause upregulation of procollagen I and decorin in vivo. To this end, reference is made to the passages under the heading "Identification of procollagen I and decorin upregulation in skin in vivo following topical retinoic acid treatment for comparative purposes" in that application.

### Example 1

### Procedure For Measuring Procollagen-I and Decorin Synthesis In Human Dermal Fibroblasts

### Preparation of Dermal Fibroblast Conditioned Medium.

Primary human foreskin fibroblasts at passage 2 (P2) were seeded into 12-well plates at 10000 cells/cm2 and maintained for 24 hours in an atmosphere of 5% carbon dioxide and 4% oxygen in Dulbeccos Modified Eagles Medium (DMEM) supplemented with 10% foetal calf serum. After this time the cells were washed with serum free DMEM and then incubated in fresh serum free DMEM for a further 60 hours. The fibroblast monolayers were then washed again with serum free DMEM. Test reagents and vehicle controls were added to the cells in triplicate in a final volume of 0.4ml/well fresh serum free DMEM and incubated for a further 24 hours. This fibroblast conditioned medium was either analysed immediately or snap frozen in liquid nitrogen and stored at -70oC for future analysis. The cells were then counted and data from the dot-blot analysis subsequently standardised to cell number.

### Dot Blot Assay for Procollagen-I and Decorin Protein in Dermal Fibroblast Conditioned Medium

Samples of conditioned medium from dermal fibroblasts treated with vehicle (as a control) or test reagents were supplement with 20mM dithiothreitol (1:10 dilution of 200mM stock solution) and 0.1% sodium dodecylsuphate (1:100 dilution of 10% stock solution), mixed well and then incubated at 75oC for 2 minutes. A standard for the assay was generated by serial dilution of neat fibroblast conditioned medium from fibroblasts seeded at 10000 cells/cm2 in a 175cm2 flask and maintained in serum free DMEM as described above.

Assay samples were subsequently applied in triplicate to a pre-wetted sheet of Immobilon-P transfer membrane using the 96-well Bio-Dot Apparatus from Bio-Rad as described in the manufacturers guidelines. Approximately 200µl of medium was applied per well. The medium was allowed to filter through the membrane under gravity (30 minutes) after which the membrane was washed twice with PBS (200µl). These PBS washes were allowed to filter through the membrane under gravity (2x15 minutes). The Bio-Dot apparatus was then attached to a vacuum manifold and a third and final PBS wash carried out under suction.

The apparatus was disassembled, the membrane removed and quickly cut as required before being placed in blocking buffer overnight at 4oC. Membranes prepared for decorin analysis were blocked with 3% (w/v) BSA/0.1% (v/v) Tween 20 in PBS, whilst those for procollagen-I analysis were blocked with 5% (w/v) non fat dried milk powder/ 0.5% Tween 20 in PBS.

The following day, the membranes were probed with 1:10000 dilution of primary antibodies to either human procollagen-I (MAB1912; rat monoclonal; Chemicon Int. Inc., Temecula, CA) or human decorin (rabbit polyclonal; Biogenesis) for 2 hours at room temperature. The membranes were subsequently washed with TBS/ 0.05% Tween 20 (3 x 5 minutes) and then incubated with 1:1000 dilution of 125 I-conjugated anti-rat or anti-rabbit F(ab')2 fragments (Amersham) as required for 1 hour at room temperature. Following this the Immobilon strips were again washed with TBS/Tween 20 (3 x 5 minutes) before being allowed to dry in air at room temperature.

The dried membranes were wrapped in cellophane and exposed to a Molecular Dynamics storage phosphor screen for 16-18 hours. At the end of this time the exposed screen was scanned by a phosphorimager (Molecular Dynamics Phosphorimager SF) using ImageQuant^{™} software. Dot intensity was assessed by computer-assisted image analysis using the quantification tools in ImageQuant^{™}, standardised to cell number and the effects of various test reagents on decorin and procollagen-I synthesis were determined relative to a vehicle treated control value of 100 arbitrary units.

Table 1 below indicates the effects of Chromdaena Odorata extract on procollagen-I and decorin synthesis in human dermal fibroblasts, and the amounts in which it was applied. In order to normalise the results the effects of the test substance was determined relative to a vehicle treated control value of 100 arbitrary units. For comparison, a trial was performed with retinoic acid to assess its effect on decorin synthesis in human dermal fibroblasts. The concentrations of reagents used in the trials had no influence on cell viability.

The extract of Chromolaena Odorata was prepared by concentrating the aqueous extract obtained from boiling Siam weed leaves in water for 30 minutes.

The extract was tested at levels of 0.1 to 1 µg/ml in an in vitro fibroblast model containing 4% oxygen, as described in above.

The model was analysed for both procollagen-I and decorin. Their results are shown below.

**Table 1**

| **Treatment** | Procollagen I | Decorin |
|---|---|---|
| Control | 100 | 100 |
| Extract of Chromolaena | | |
| Odorata at: | | |
| 0.01 µg/ml | - | 172 |
| 0.1 µg/ml | 113 | 180 |
| 1.0 µg/ml | 116 | 182 |

The results indicate that decorin synthesis in the model is substantially up regulated by even relatively low levels (e.g. 0.01 µg/ml) of extract. Small but positive effects were also seen on procollagen I synthesis, as compared to the control. The 1.0 µg/ml sample was also comparatively tested with the retinoic acid (1µm), showing an upregulation of decorin, 138 ± 14.0 (p = 0.035, n = 4), as determined relative to a vehicle treated control value of 100 arbitrary units. This further indicates the magnitude of the increase of decorin synthesis in human dermal fibroplasts effected by Chromolaena Odorata extract, compared to the effect of retinoic acid.

### Example 2

The formulation below describes an oil in water cream suitable for the methods and uses according to the present invention. The percentages indicated are by weight of the composition.

| | wt% |
|---|---|
| Mineral Oil | 4 |
| Chromolaena Odorata extract | 1.15 |
| Brij 56* | 4 |
| Alfol 16RD** | 4 |
| Triethanolamine | 0.75 |
| Butane-1,3-diol | 3 |
| Xanthan gum | 0.3 |
| Perfume | qs |
| Butylated hydroxy toluene | 0.01 |
| Water | to 100 |

| | |
|---|---|
| ^{*}Brij 56 is cetyl alcohol POE (10) ^{**} Alfol 16RD is cetyl alcohol | |

### Example 3

The formulation below describes an emulsion cream according to the present invention.

| FULL CHEMICAL NAME OR CTFA NAME | TRADE NAME | WT. % |
|---|---|---|
| Chromolaena Odorata extract | | 2.0 |
| Disodium EDTA | Sequesterene Na2 | 0.05 |
| Magnesium aluminium silicate | Veegum Ultra | 0.6 |
| Methyl paraben | Methyl Paraben | 0.15 |
| Simethicone | DC Antifoam Emulsion | 0.01 |
| Butylene glycol 1,3 | Butylene Glycol 1,3 | 3.0 |
| Hydroxyethylcellulose | Natrosol 250HHR | 0.5 |
| Glycerine, USP | Glycerine USP | 2.0 |
| Xanthan gum | Keltrol 1000 | 0.2 |
| Triethanolamine | Triethanolamine (99%) | 1.2 |
| Stearic acid | Pristerene 4911 | 3.0 |
| Propyl paraben NF | Propylparaben NF | 0.1 |
| Glyceryl hydrostearate | Naturechem GMHS | 1.5 |
| Stearyl alcohol | Lanette 18 DEO | 1.5 |
| Isostearyl palmitate | Protachem ISP | 6.0 |
| C12-15 alcohols octanoate | Hetester FAO | 3.0 |
| Dimethicone | Silicone Fluid 200 (50cts) | 1.0 |
| Cholesterol NF | Cholesterol NF | 0.5 |
| Sorbitan stearate | Sorbitan Stearate | 1.0 |
| Butylated hydroxytoluene | Embanox BHT | 0.05 |
| Tocopheryl acetate | Vitamin E Acetate | 0.1 |
| PEG-100 stearate | Myrj 59 | 2.0 |
| Sodium stearoyl lactylate | Pationic SSL | 0.5 |
| Hydroxycaprylic acid | Hydroxycaprylic Acid | 0.1 |
| Retinyl palmitate | Vitamin A Palmitate | 0.06 |
| Alpha-bisabolol | Alpha-bisabolol | 0.2 |
| Water, DI | | q.s. to 100 |

Both the above topical compositions of example 2 and 3 provide an effective cosmetic treatment to improve the appearance of wrinkled, aged and/or photo-damaged skin, when applied to skin that has deteriorated through the aging or photoageing or when applied to youthful skin to help prevent or delay such deteriorative changes. The compositions can be processed in conventional manner

## Claims

1. A cosmetic method of treating and preventing normal skin conditions due to chrono-ageing or photo-ageing comprising applying to the skin a topical composition comprising an effective amount of an aqueous extract of Chromolaena Odorata.

2. A cosmetic method according to claim 1 wherein the normal skin conditions comprise wrinkles, lines, sagging, hyper pigmentation and age spots.

3. A cosmetic method according to claim 1 or claim 2 wherein the extract is present at a level of 0.0001% to 1% by weight of the composition.

## Patentansprüche

1. Kosmetisches Verfahren zum Behandeln und Vorbeugen von normalen Hautzuständen aufgrund von Zeitalterung oder Lichtalterung, umfassend das Auftragen einer topischen Zusammensetzung, die eine wirksame Menge eines wässerigen Extrakts von Chromolaena Odorata umfaßt, auf die Haut.

2. Kosmetisches Verfahren nach Anspruch 1, wobei die normalen Hautzustände Falten, Linien, Durchhängen, Hyperpigmentierung und Altersflecken umfassen.

3. Kosmetisches Verfahren nach Anspruch 1 oder Anspruch 2, wobei das Extrakt bei einem Gehalt von 0,0001 bis 1 Gew.-% der Zusammensetzung vorliegt.

## Revendications

1. Procédé cosmétique de traitement et de prévention de conditions normales de la peau dues au chrono-vieillissement ou au photo-vieillissement comprenant l'application sur la peau d'une composition topique comprenant une quantité efficace d'un extrait aqueux de Chromolaena Odorata.

2. Procédé cosmétique selon la revendication 1 dans lequel les conditions normales de la peau comprennent les rides, les lignes, l'affaissement, l'hyperpigmentation et les taches de vieillissement.

3. Procédé cosmétique selon la revendication 1 ou la revendication 2 dans lequel l'extrait est présent à un taux de 0,0001 % à 1 % en poids de la composition.
